# EUROPÄISCHE PATENTSCHRIFT

(11) **EP 1 934 574 B1**
(45) Veröffentlichungstag und Bekanntmachung des Hinweises auf die Patenterteilung: **04.02.2009**
(21) Anmeldenummer: 06806271.0
(22) Anmeldetag: 13.10.2006
(51) Int. Cl.: G01N 1/10

(54) **PROBENAHMESYSTEM FÜR FLUIDE PROBEN**
SAMPLING SYSTEM FOR FLUID SAMPLES
SYSTEME DE PRELEVEMENT D'ECHANTILLONS POUR DES ECHANTILLONS FLUIDES

(30) Priorität: 14.10.2005 DE 102005049226
(43) Veröffentlichungstag der Anmeldung: 25.06.2008
(73) Patentinhaber: Technische Universität München, 80333 München (DE)
(72) Erfinder: HILLER, Julia, 90455 Nürnberg (DE); ZACHER, Karl-Heinz, 85656 Buch am Buchrain (DE); WEUSTER-BOTZ, Dirk, 85221 Dachau (DE); KUSTERER, Andreas, 85586 Poing (DE)
(74) Vertreter: Pfenning, Meinig & Partner GbR
(86) Internationale Anmeldenummer: PCT/EP2006/009928
(87) Internationale Veröffentlichungsnummer: WO 2007/042323

(56) Entgegenhaltungen:
- WO-A2-20/04087859
- THEOBALD, U. ET AL.: "In Vivo Analysis of Glucose-Induced Fast Changes in Yeast Adenine Nucleotide Pool Applying a Rapid Sampling Technique" ANALYTICAL BIOCHEMISTRY, Bd. 214, Nr. 31-37, 1993, XP002421327

## Beschreibung

Die vorliegende Erfindung betrifft ein Probenahmesystem für fluide Proben. Derartige Probenahmesysteme werden insbesondere benötigt, wenn eine Probe aus einer Flüssigkeit oder einem Gasvolumen entnommen werden soll. Derartige Probenahmesysteme werden insbesondere benötigt, um in Bioreaktoren den Verfahrensablauf zu überwachen, also insbesondere im Bereich der Biotechnologie, jedoch auch der Chemieindustrie, der Pharmaindustrie, der Lebensmittelindustrie sowie im Bereich der Umwelttechnik und bei Behörden, beispielsweise Umweltschutzbehörden oder Analyselaboren.

Zur manuellen Probenahme werden bei Laborbioreaktoren aus Edelstahl üblicherweise Hähne und Ventile im Boden des Laborbioreaktors eingesetzt. Bei im Labormaßstab häufig verwendeten Glasreaktoren, die nur von oben zugänglich sind, wird die manuelle Probenahme normalerweise mit Hilfe eines sog. Tauchrohres durchgeführt. Hierbei wird die Probe mit einer spritze und unter Einsatz einer Pumpe über ein Rohr aus dem Bioreaktor herausbefördert. Diese Verfahrensweise hat zur Folge, dass die Probe aus dem Reaktor üblicherweise von oben über ein Tauchrohr gezogen wird. Wird die Probe lediglich an der Oberfläche des Reaktorvolumens gezogen, so können verfälschte Probenahmen entstehen. Reicht das Tauchrohr jedoch sehr weit in das Volumen ein, so entsteht ein relativ großes Totvolumen, das ebenfalls ein Verfälschung der Probe bewirken kann oder einen großen Probenverlust bedeutet, da das Totvolumen gewöhnlich dann verworfen werden muss.

Die WO 2004/087859 A2 beschreibt ein Probenahmesystem für fluide Proben mit einer Probenahmesonde zum Eintauchen in ein Fluidvolumen und zur Entnahme einer fluiden Probe aus dem Fluidvolumen, wobei die Probenahmesonde und das Probenaufnahmegefäß in ihrer Wandung jeweils ein erstes und ein zweites Ventil aufweisen, die derart ausgebildet sind, dass sie sich in Kontakt miteinander öffnen.

In U. Theobald et al., Analytical Biochemistry 214, 31-37 (1993) wird ein Probenahmesystem für die Entnahme von fluiden Proben aus einem Fermenter beschrieben, bei welchem eine erste Nadel in einem Fermenter eingeführt wird und eine zweite Nadel in ein mit einer Membran verschlossenes Reagenzglas eingestochen wird. Die beiden Nadeln sind über ein manuell betätigbares Ventil miteinander verbunden. Zur Entnahme der Probe aus dem Fermenter kann das Ventil geöffnet werden und nach der Probenentnahme von Hand wieder geschlossen werden.

Aufgabe der vorliegenden Erfindung ist es daher, ein sicheres Probenahmesystem zur Verfügung zu stellen, das eine totvolumenarme manuelle Probenentnahme ermöglicht.

Diese Aufgabe wird durch das Probenahmesystem nach Anspruch 1 gelöst. Vorteilhafte Weiterbildungen des erfindungsgemäßen Probenahmesystems ergeben sich aus den weiteren abhängigen Ansprüchen.

Erfindungsgemäß weist das Probenahmesystem, das sämtliche Proben, insbesondere für Gase und Flüssigkeiten, verwenden kann, drei einzelne Elemente auf, die jedoch aufeinander abgestimmt sind. Als erstes wird in den Bioreaktor eine Probensonde eingesetzt, die als Hohlvolumen, insbesondere als Hohlrohr, ausgebildet sein kann. Diese Probensonde ist an ihrem einen Ende offen und an ihrem anderen in den Reaktor ragenden Ende über ein Ventil verschlossen. Das Ventil besitzt eine Hohlnadel, über die die Außenseite des Ventils mit der Innenseite des Ventils verbunden wird, wenn das Ventil geöffnet wird.

Weiterhin ist ein Probeaufnahmegefäß vorgesehen, das der Aufnahme der gezogenen Probe dient. Dieses ist mit einem Septum verschlossen und so dimensioniert, dass es in die Probensonde eingeführt werden kann. Wird es weit genug in die Probensonde eingeführt, so wird das an seinem einen Ende befindliche Septum von der Hohlnadel durchstochen. Beim weiteren Einführen in die Probensonde drückt das Probenaufnahmegefäß gegen das Ventil und öffnet so mechanisch das Ventil. Der genaue Mechanismus zur Öffnung des Ventils wird später beschrieben. Auf diese Weise wird eine Öffnung zwischen der Außenseite der Probensonde und dem Innenvolumen des Probenaufnahmegefäßes freigegeben. Ist das Probenaufnahmegefäß zumindest teilweise evakuiert, wird nun die Probe aus dem zu beprobenden Fluidvolumen über das Ventil und die Hohlnadel in das Innenvolumen des Probenaufnahmegefäßes gesaugt.

Als drittes Element ist eine Halterung für das Probenaufnahmegefäß vorgesehen, in die das Probenaufnahmegefäß eingeführt werden kann. Durch diese Halterung ist eine definierte Einführung des Probenaufnahmegefäßes in die Probensonde möglich.

Das erfindungsgemäße Probenahmesystem ermöglicht es, reproduzierbar und zuverlässig Proben aus einem Fluidvolumen zu entnehmen. Es ist sicher und durch geschultes Personal auf einfache Weise anzuwenden.

Da als Probenaufnahmegefäß herkömmliche standardisierte Probenaufnahmegefäße, beispielsweise sog. Vacutainer der Fa. BD GmbH, verwendet werden können, können die laufenden Kosten für das erfindungsgemäße Probenahmesystem gering gehalten werden. Insbesondere die Kosten für die Verbrauchsartikel, wie Probenaufnahmegefäße, sind durch die Verwendung standardisierter, kommerzieller Produkte günstig zu halten.

Das erfindungsgemäße Probenahmesystem hat weiterhin den Vorteil, dass das Totvolumen, nämlich das Durchgangsvolumen des Ventils und der Hohlnadel, gering ist. Dennoch ist über die Probensonde eine Entnahme aus einem Reaktor, insbesondere auch aus einem Glasreaktor, möglich.

Das System ist also bei jeder Art von Bioreaktoren, insbesondere auch bei kleinen oder mittelgroßen Glaslaborreaktoren, einsetzbar. Durch den durchgehend gewährleisteten Abschluss der zu ziehenden Probe und des zu beprobenden Fluides von der Außenwelt ist auch eine Probenentnahme toxischer oder gesundheitsgefährdender Stoffe ohne weitere Schutzvorkehrungen bzw. mit geringen Schutzvorkehrungen möglich.

Das Probenaufnahmegefäß kann weiterhin mit Reagenzien gefüllt werden, um beispielsweise unerwünschte Reaktionen in der Probe nach der Probenentnahme zu unterbinden.

Es ist weiterhin möglich, ein Probenaufnahmegefäß mit einer Sterilisierungslösung zu befüllen und dieses Probenaufnahmegefäß in die Probensonde einzuführen, nachdem bzw. bevor eine Probe gezogen wird. Hierdurch ist es möglich, die Hohlnadel unmittelbar nach der Probennahme, vor einer Probennahme oder auch während des gesamten Zeitraumes zwischen zwei Probennahmen steril zu halten.

Die Probensonde ist vorteilhafterweise ein nach oben offenes Edelstahlrohr, das an seinem unteren Ende mit dem Ventil verschlossen ist. Sein Außendurchmesser sollte so gewählt werden, dass es in den Standardstutzen eines Laborreaktors eingeführt werden kann. Die Probensonde kann weiterhin eine Feder aufweisen, die bei Einführen des Probenaufnahmegefäßes von diesem zusammengedrückt wird, sodass eine Kraft auf das Probenaufnahmegefäß ausgeübt wird, das dieses nach erfolgter Probennahme wieder aus der Probensonde herausschiebt. Dadurch wird die Entnahme des Probenaufnahmegefäßes aus der Probensonde zusätzlich unterstützt.

Auch die Halterung für das Probenaufnahmegefäß kann als Hohlrohr ausgeführt sein, dessen Innendurchmesser gleich oder geringfügig größer als der Außendurchmesser des Probenaufnahmegefäßes ist. Dies muss zumindest für einen Teil des Probenaufnahmegefäßes gelten, da es lediglich erforderlich ist, das Probenaufnahmegefäß teilweise in die Halterung einzuführen. Die Halterung kann weiterhin vorteilhafterweise ein Griffelement aufweisen, um eine einfache Bedienung zu ermöglichen. Dieses kann sich in Längsrichtung an das Hohlrohr anschließen, wobei der Durchmesser des Griffelementes auch größer oder kleiner sein kann als der Außendurchmesser des Hohlrohres. In letzterem Falle ist dann ein konischer Übergang zwischen dem Hohlrohr und dem Griffelement von Vorteil.

Die Halterung kann weiterhin Federelemente aufweisen, die das Pröbenaufnahmegefäß in der Halterung festlegen. Dies kann beispielsweise über Blattfedern erfolgen, die an der Außenseite des Hohlrohres angeordnet sind und mit ihrem freien Ende bzw. einem freien Bereich durch eine Öffnung in dem Hohlrohr auf ein eingeführtes Probenaufnahmegefäß drücken. In diesem Falle werden die Blattfedern weiter zusammengedrückt und das Probenaufnahmegefäß noch fester gehalten, wenn die Halterung in die Probensonde eingeführt wird. Zwischen den auf das Probenaufnahmegefäß drückenden Bereichen der Blattfedern und dem Probenaufnahmegefäß selbst kann ein O-Ring, der sich an der Innenseite des Hohlrohres der Halterung befindet oder in eine umlaufende Nut in der Wandung des Hohlrohres der Halterung eingefügt ist, befinden. Dieses verbessert weiterhin die Haltekraft und verringert das Risiko einer Beschädigung des Probenaufnahmegefäßes durch die federnden Elemente.

In einer weiteren vorteilhaften Ausführungsform weist die Halterung einen Zapfen bzw. einen Stift auf, der nach außen vorragt. Zu diesem Stift ist eine entsprechende Nut in der Probensonde eingefügt, sodass die Halterung mit dem Stift derart in die Probensonde eingeführt werden kann, dass der Stift in der Nut gleitet. Die Nut kann eine erste Stufe ähnlich einem Bajonettverschluss aufweisen, bei dem eine weitere Einführung der Halterung in die Probensonde erst nach einer Drehung der Halterung möglich ist. Weiterhin kann sie einen Endanschlag aufweisen, der die Position der Halterung in der Probensonde festlegt, bei dem die Halterung maximal weit in die Probensonde eingeführt ist.

Die erste Stufe der Nut kann nun so ausgelegt sein, dass die Halterung mit dem Stift lediglich so weit in die Probensonde eingeführt ist, dass das Septum, dass das Probenaufnahmegefäß verschließt, gerade eben durch die Nadel des Ventils durchstochen ist, jedoch das Ventil noch geschlossen ist. Diese Position eignet sich insbesondere, um die Hohlnadel mit einem Probenaufnahmegefäß, das ein Sterilisierungsmittel enthält, zu sterilisieren bzw. in dieser Position steril zu halten.

Nach Drehen der Halterung und weiterer Einführung der Halterung in die Probensonde übt dann die Halterung oder das Probenaufnahmegefäß eine Kraft auf das Ventil aus, sodass das Ventil geöffnet wird und nun eine Probe über das Ventil und die Hohlnadel in das Probenaufnahmegefäß gezogen wird. Dies ist die Position der Halterung, bei der eine Probennahme erfolgt.

Erfindungsgemäß ist es selbstverständlich auch möglich, die entsprechende Nut an der Außenseite der Halterung anzuordnen und die Probensonde mit einem nach innen vorstehenden Stift zu versehen. Damit kann dieselbe Wirkung erzielt werden, wie durch einen Stift an der Halterung und eine gestufte Nut an der Probensonde.

Im Folgenden werden nun Beispiele erfindungsgemäßer Probenahmesysteme gegeben. Es zeigen
Figur 1 ein Probenahmesystem in einem Bioreaktor;
Figur 2 eine Probennahmesonde im Schnitt (Figur 2A) und in Vorderansicht (Figur 2B), wobei Figur 2A einen Schnitt längs der Linie A-A in Figur 2B darstellt;
Figur 3 eine vergrößerte Ansicht des mit einem Ventil verschlossenen Endes der Probennahmesonde aus Figur 2;
Figur 4 ein Probenaufnahmegefäß;
Figur 5 ein Probenaufnahmegefäß in verschiedenen Positionen innerhalb einer Probensonde;
Figur 6 drei verschiedene Ansichten bzw. Positionen einer Halterung;
Figur 7 ein weiteres Beispiel für ein erfindungsgemäßes Probenahmesystem.
Figur 8 zeigt ein erfindungsgemäßes Probennahmesystem, in welchem das Probengefäß in einer Halteklammer gehalten wird.
Figur 9 zeigt ein Probennahmegefäß.
Figur 10 zeigt den Schnitt durch eine erfindungsgemäße Halteklammer.
Figur 11 zeigt ein erfindungsgemäßes Ventil mit einer Kanüle.
Figur 12 zeigt den Schnitt durch ein erfindungsgemäßes Ventil mit einer in einem Septum untergebrachten Nadelspitze.
Figur 13 zeigt ein Ventil entsprechend Figur 12, welches in einer Probennahmesonde untergebracht ist.
Figur 14 zeigt ein Ventil entsprechend Figur 12, welches in einer Probennahmesonde untergebracht ist und sich im geöffneten Zustand befindet.
Figur 15 zeigt eine abdichtende Verschraubung der erfindungsgemäßen Probennahmesonde.

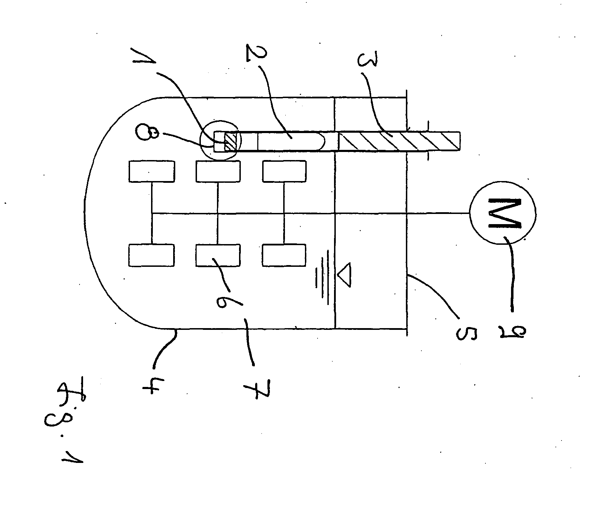

Figur 1 zeigt die Konstruktions- und Funktionsweise eines erfindungsgemäßen Probenahmesystems am Beispiel eines Laborbioreaktors 4. In dem Laborbioreaktor 4, der durch einen Deckel 5 verschlossen ist, befindet sich eine Zellsuspension 7, die durch einen Rührer 6 in Suspension gehalten wird. In diese Zellsuspension 7 taucht nun ein Probenahmesystem ein, das aus insgesamt drei Elementen besteht. Dies sind zum einen eine Probensonde 1, die in den Reaktorverschluss bzw. - deckel 5 eingesetzt ist. Sie ist so dimensioniert, dass sie in einen Standardstutzen 8 des Reaktors 4 eingeführt werden kann.

Die Probensonde selbst ist ein oben offenes Edelstahlrohr, an dessen unterem Ende sich ein Probenahmeventil befindet.

Die Probensonde taucht nun in die Zellsuspension 7 ein und ermöglicht so an ihrem unteren Ende eine Probenahmeposition, die weitgehend im Inneren des Reaktors 4 liegt. Dadurch wird zum einen die Probe unmittelbar im Inneren der Zellsuspension 7 entnommen, und zum anderen der Transport der Probe aus der Zellsuspension 7 in ein Probenaufnahmegefäß 2 minimiert. Dadurch kann sichergestellt werden, dass zum einen die Verweilzeit während der Probennahme bis zum Erreichen des Probenaufnahmegefäßes 2 kurz gehalten wird und zum anderen die Totvolumina dieses Weges nur gering sind.

Der Rührer 6 wird durch einen Motor 9 angetrieben und hält die Zellsuspension in Bewegung.

Innerhalb der Probensonde 1 befindet sich das Probenaufnahmegefäß 2, sowie eine Halterung 3 für das Probenaufnahmegefäß 2.

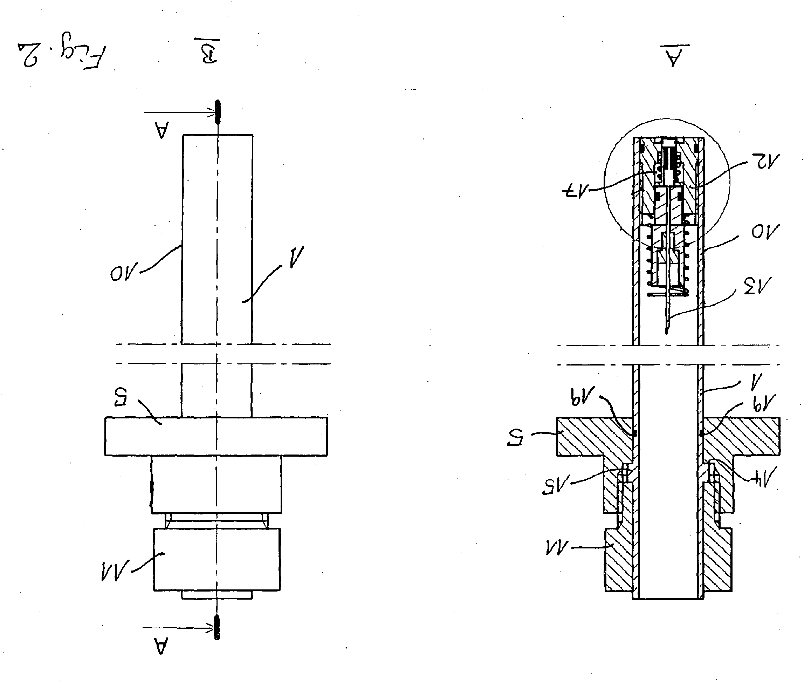

Figur 2 zeigt nun eine erfindungsgemäße Probensonde 1 mit einem Edelstahlrohr 10. Figur 2B zeigt eine Aufsicht auf die Anordnung der Probensonde 1 in dem Reaktorverschluss 5 während Figur 2A einen Schnitt durch diese Anordnung längs der Linie A-A in Figur 2B darstellt. Hier wie im Folgenden werden gleiche bzw. ähnliche Elemente mit gleichen bzw. ähnlichen Bezugszeichen versehen. Das Edelstahlrohr 10 der Probensonde 1 ist in dem Reaktordeckel 5 in den Standardstutzen eingelassen und mit einer Überwurfmutter 11 gesichert. Das Edelstahlrohr 10 weist weiterhin einen Flansch 15 auf, der im Eingriff ist mit einem Flansch 14 in dem Reaktorverschluss 5. Durch diese beiden Flansche 14 und 15 ist die Eintauchtiefe des Edelstahlrohres 10 festgelegt. Zusätzlich ist in einer außenliegenden umlaufenden Nut in dem Edelstahlrohr auf Höhe des Reaktorverschlusses 5 ein O-Ring 19 angeordnet, der zwischen dem Verschluß 9 und dem Edelstahlrohr 10 abdichtet.

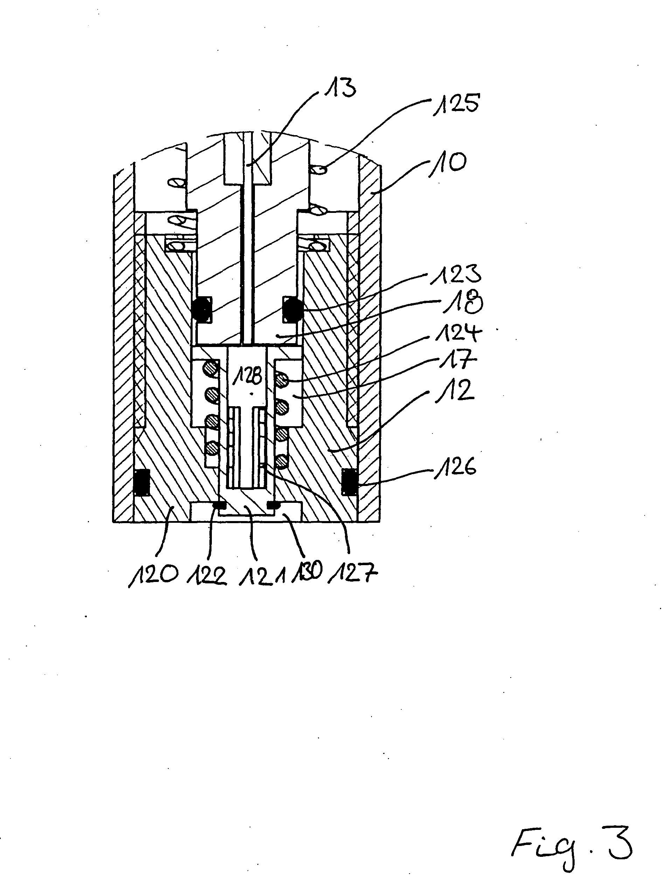

In dem Edelstahlrohr 10 ist an seinem unteren Ende ein Ventil 12 mit einem Ventilkörper 120 abdichtend angeordnet. Der Ventilkörper 120 besitzt eine durchgehende Bohrung 17 in Längsrichtung der Probensonde 1. In diese Bohrung 17 ist, wie in Figur 3 in vergrößerter Darstellung zu erkennen ist, ein Ventilzylinder 18 eingebracht, der seinerseits an den Wänden der Bohrung 17 dichtend anliegt. Diese Abdichtung wird zusätzlich über einen O-Ring 123 verbessert.

Der Ventilzylinder 18 ist über die Feder 124 in dem Ventilkörper 120 federnd gelagert und kann aus der in Figur 3 gezeigten Position nach unten gegen die Kraft der Feder 124 in Längsrichtung der Probensonde 1 verschoben werden.

Der Ventilkörper 120 besitzt am Ende der Probensonde 1 eine Aussparung 130, an der der Ventilzylinder 18 hervorsteht. Dort ist der Ventilzylinder 18 mit einer umlaufenden Gummiringdichtung 122 versehen, die den Spalt zwischen dem Ventilzylinder 18 und dem Ventilkörper 120 abdichtet, wenn der Ventilzylinder 18 durch die Feder 124 maximal in das Innere der Probensonde 1 verschoben wird.

Der Ventilzylinder 18 besitzt weiterhin seinerseits eine Innenbohrung 128. Ausgehend von dieser Innenbohrung 128 befinden sich in den Seitenwänden des Ventilzylinders 18 Durchlassöffnungen 127, die die Außenseite der Wandung des Ventilzylinders 18 mit der Bohrung 128 verbinden.

Wird nun der Ventilzylinder 18 gegen die Federkraft der Feder 124 nach unten gedrückt, so werden die Bohrungen 127 offen gelegt und ein Fluid kann von außerhalb der Probensonde 1 in die Bohrung 127 und die Bohrung 128 einströmen.

Wie in den Figuren 2 und 3 zu erkennen ist, ist in dem Ventilzylinder 18 weiterhin eine Hohlnadel 13 angeordnet, die mit der Bohrung 128 und damit mit dem Innenvolumen des Ventilzylinders 18 kommuniziert. Diese Hohlnadel 13 ist in Längsrichtung der Probensonde 1 orientiert und steht an ihrem dem Ventilzylinder 18 abgewandten Ende über den Ventilzylinder 18 vor.

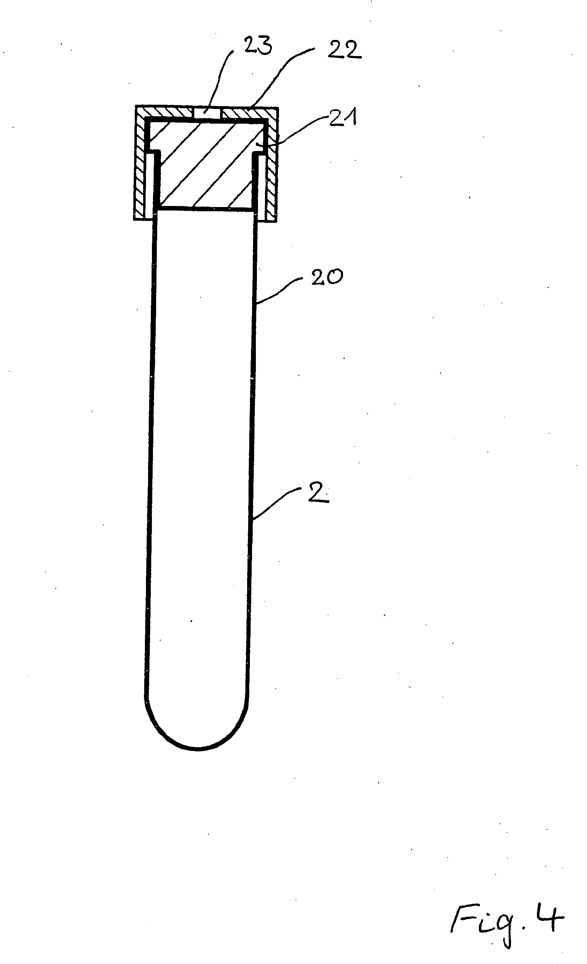

Figur 4 zeigt ein erfindungsgemäßes Probenaufnahmegefäß 2. Dieses besteht im Wesentlichen aus einem Reagenzglas 20 aus Glas oder Kunststoff, das mit einem Septum 21 verschlossen ist. Über dem Septum 21, im vorliegenden Beispiel aus Silikon, ist weiterhin eine Schutzkappe 22 angeordnet, die jedoch in der Mittelachse des Reagenzglases 20 eine Öffnung 23 aufweist. Durch diese Öffnung 23 kann die Hohlnadel 13 im Anwendungsfall das Septum 21 durchstechen.

Als Probenaufnahmegefäße 2 eigenen sich insbesondere herkömmliche bereits im Handel erhältliche sog. "Vacutainer", die sowohl leer als auch mit unterschiedlichen Puffern und Reagenzien befüllt erhältlich sind. Sie werden üblicherweise im medizinischen Bereich zur Aufbereitung von Blutproben verwendet. Daher ist auch üblicherweise bereits der Inhalt dieses Gefäßes durch die Farbe der Schutzkappe 22 und des Septums 21 eindeutig gekennzeichnet und jedes Gefäß 2 bereits mit einem Beschriftungsfeld versehen.

Derartige Vacutainer sind in unterschiedlichen Volumina erhältlich und sehr kostengünstig. Diese genormten Probenaufnahmegefäße ermöglichen den Einsatz der vorliegenden Erfindung bei Routinekontrollen von Prozessen und Anlagen, beispielsweise auch durch das Schichtpersonal oder auch durch Behörden. Die oftmals aufwendige und qualifiziertes Personal erfordernde Analytik kann erst später in entsprechend qualifizierten und zertifizierten Labors erfolgen.

Von besonderem Vorteil ist die geschlossene Konstruktion des Probenaufnahmegefäßes 2, da eine Manipulation bei der Probennahme und dem anschließenden Transport zur Analyse mit einfachen Maßnahmen ausgeschlossen werden kann. Auch können aufgrund der geschlossenen Konstruktion gesundheitsgefährdende bzw. toxische Proben aus Apparaten, Anlagen oder Kanälen entnommen werden, ohne das Personal zu gefährden.

Im nicht-zertifizierten Laborbereich ist es auch möglich, die vorhandenen kommerziell schon erhältlichen Probenaufnahmegefäße 2 mit Hilfe einer Spritze mit eigenen Reagenzien zu befüllen, z.B. können die Gefäße 2 mit kaltem Methanol befüllt werden, um bei der Probennahme die in der Probe stattfindenden biologischen Reaktionen sofort zu stoppen.

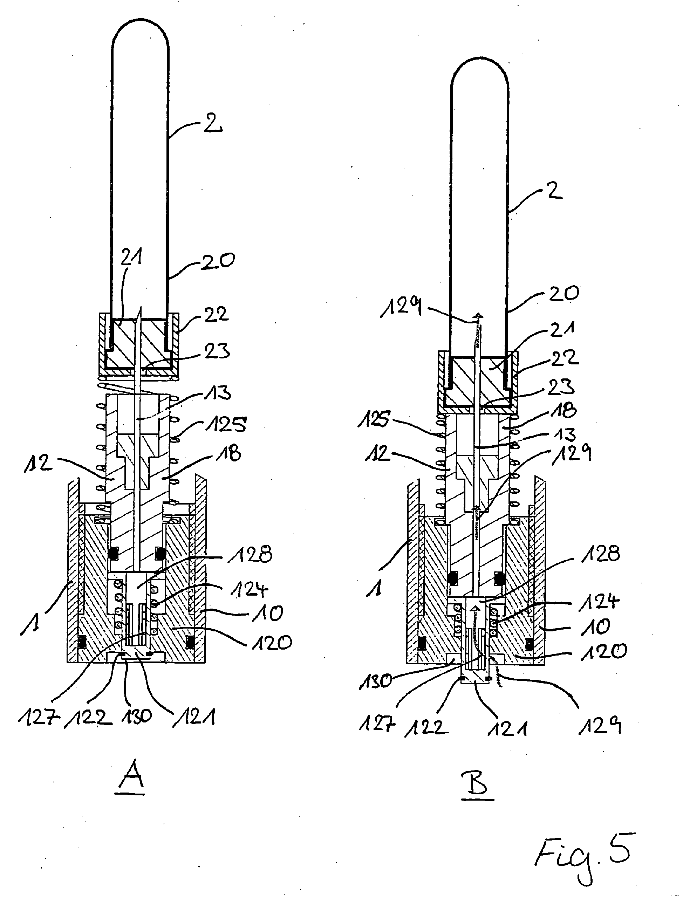

Figur 5 zeigt nun ein Probenaufnahmegefäß 2 in zwei verschiedenen Anordnungen relativ zu dem Ventil 12 einer Probensonde 1. In Figur 5A ist dabei eine Position dargestellt, bei der das Probenaufnahmegefäß die Rückstellfeder 125, die den Ventilzylinder 18 umgibt, noch nicht zusammendrückt. Da die Hohlnadel 13 jedoch ausreichend weit aus dem Ventilzylinder 18 hervorsteht, durchsticht sie bereits das Septum 21 durch die Öffnung 23. Auf diese Weise ist damit bereits ein Kontakt zwischen dem Inneren der Hohlnadel 13 und dem Inneren des Probenaufnahmegefäßes 2 hergestellt. Ist das Probenaufnahmegefäß 2 mit einer Sterilisierungslösung beladen, kann so die Nadel 13 sterilisiert werden bzw. dauerhaft steril gehalten werden.

Die Nadel 13 kann ihrerseits eine Hülle (hier nicht dargestellt) besitzen, die sie üblicherweise bedeckt und die beim Aufsetzen des Probenaufnahmegefäßes 2 zurückgedrückt wird und damit die Spitze der Hohlnadel 13 freigibt.

Figur 5B zeigt eine weitere Position, bei der die Schutzkappe 22 des Probenaufnahmegefäßes 2 auf den Ventilzylinder 18 drückt und auf diese Weise den vordersten Teil 121 des Ventilzylinders 18 aus der Ausnehmung 130 herausdrückt. Dadurch wird der Gummiring 122 aus seinem Sitz gedrückt und die Öffnungen 127 treten in Kontakt mit dem Außenraum der Probensonde 1. Die Pfeile 129 zeigen nun einen möglichen Strömungsweg für das zu beprobende Fluid durch die Öffnung 127, das Innenvolumen 128 des Ventilzylinders 18 sowie das Innenvolumen der Hohlnadel 13 in den Innenraum des Probenaufnahmegefäßes 2. Dabei übt die Feder 125 eine Rückstellkraft auf das Probenaufnahmegefäß 2 aus, die jedoch vom Bediener während der Probennahme überwunden wird.

Auch die Feder 124 wird durch den Ventilzylinder 18 zusammengedrückt und übt so eine Rückkraft auf den Ventilzylinder 18 aus. Bei nachlassendem Druck des Probenaufnahmegefäßes 2 auf den Ventilzylinder 18 wird so zuerst der Ventilstift 121 wieder in seinen abdichtenden Sitz zurückgeschoben und erst danach das Probenaufnahmegefäß 2 von der Hohlnadel 13 gezogen.

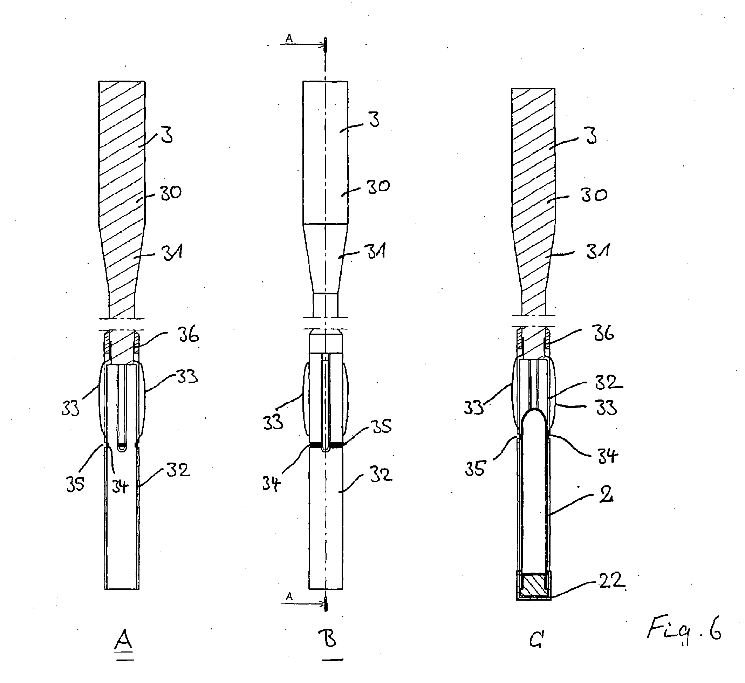

Figur 6 zeigt eine Halterung eines erfindungsgemäßen Probenahmesystems. Diese Halterung 3 ist in Figur 6B in Aufsicht und in Figur 6A in einer Schnittansicht längs des Schnittes A-A in Figur 6B dargestellt. Die Halterung 3 weist ein Griffelement 30 auf, das sich über einen konischen Bereich verengt und in einen Hohlzylinder 32 übergeht. Dieser Hohlzylinder 32 ist so gewählt, dass in ihn das Probenaufnahmegefäß 2 eingefügt werden kann. An der Außenseite des Hohlzylinders 32 sind Blattfedern 33 angeordnet, die sich in axialer Richtung des Hohlrohres 32 erstrecken. Diese Blattfedern durchgreifen das Hohlrohr 32 an Aussparungen (Nuten) 35 und drücken mit dem hier freien Ende auf einen Gummi-O-Ring 34, der innerhalb der Wandung des Hohlrohres 32 angeordnet ist.

Ist ein Probenaufnahmegefäß 2 in en Hohlzylinder 32 eingeführt (Figur 6C), so drücken die Blattfedern 33 über den O-Ring 34 auf das Probenaufnahmegefäß 2 und halten es in seiner Position fest. Damit ist das Probenaufnahmegefäß 2 in der Halterung 3 fixiert.

Soll eine Probe aus dem Bioreaktor 4 entnommen werden, so wird zuerst das Probenaufnahmegefäß 2 in die Halterung 3 eingesteckt. Die Halterung 3 wird dann in die Probensonde 1 eingeführt, wobei die an der Halterung 3 befindlichen Blattfedern 33 gequetscht werden. Sie üben hierdurch in der Probensonde 1 einen noch stärkeren Druck auf das Probenaufnahmegefäß 2 aus. Bei weiterem Einführen der Halterung 3 in die Probensonde 1 durchsticht die ventilseitige Hohlnadel 13 das Septum 21. Durch weiteren Druck auf Halterung 3 und damit auf das Probenaufnahmegefäß 2 wird die Feder 125 komprimiert und der Ventilzylinder 18 nach unten bewegt. Damit wird dann der Dichtring 122 von seinem Dichtsitz entfernt und die Öffnungen 127 im Ventilzylinder 18 freigegeben. Damit ist das Ventil in der Probensonde 1 geöffnet und die Probe wird durch die Druckdifferenz zwischen dem Probenaufnahmegefäß 2 und dem Innenraum des Reaktors 4 bis zum Druckausgleich in das Probenaufnahmegefäß 2 gefördert.

Wird das Probenaufnahmegefäß 2 durch Zug an dem Griffelement 30 der Halterung 3 wieder aus der Probensonde 1 entfernt, so wird zunächst durch die Federkraft der Feder 124 das Ventil in der Probensonde 1 geschlossen und anschließend unter Unterstützung durch die Feder 125 das Probenaufnahmegefäß 2 aus der Probensonde 1 herausgeschoben, wobei die Verbindung zwischen der Hohlnadel 13 und dem Septum 21 getrennt wird. Das Septum 21 schließt sich dann wieder.

Außerhalb der Probensonde 1 üben die Blattfedern 33 der Halterung 3 nur noch leichten Druck auf das Probenaufnahmegefäß 2 aus, so dass das nun befüllte Probenaufnahmegefäß 2 einfach aus der Halterung 3 entfernt werden kann.

Die Probe kann dann zur Weiterverarbeitung verwendet werden, insbesondere kann sie bei Verwendung eines geeigneten Zentrifugeneinsatzes unmittelbar im Probenaufnahmegefäß 2 abzentrifugiert werden.

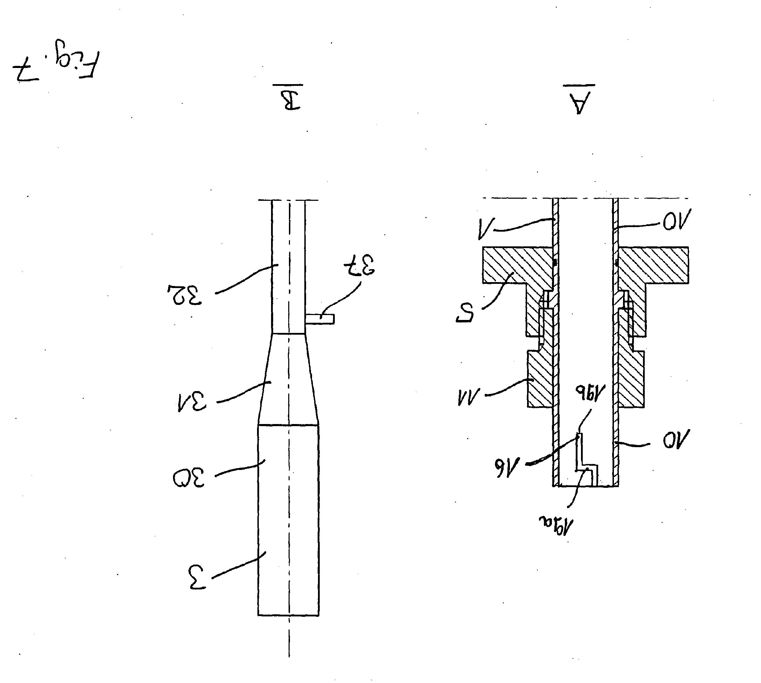

Figur 7 zeigt eine besondere Ausführungsform der Halterung 3 und der Probensonde 1, mit der eine Kontamination des Bioreaktors auf einfache Weise sicher ausgeschlossen werden kann. Hierbei ist die Probensonde 1 mit einer Nut 16 versehen. Diese Nut ist als gestufte Nut 16 in die Innenwandung der Probensonde 1 eingebracht. Sie weist eine erste Stufe 19a sowie einen Endanschlag 19b auf. In diese Nut 16 kann ein Stift 37 eingeführt werden, der an der Halterung 3 angeordnet ist. Wird der Stift 37 nun in die Nut 16 eingeführt, so erfolgt zuerst eine geführte axiale Bewegung der Halterung 3. Bei Erreichen der Stufe 19a ist dann die Halterung 3 zu drehen, um den Stift anschließend weiter längs der Nut zu führen, um dann bis zum Anschlag 19b am Ende der Nut 16 wiederum eine axiale Bewegung der Halterung 3 auszuführen.

Soll das Probenahmesystem nun zwischen den Probennahmen steril gehalten werden, so wird die Halterung 3 mit einem mit Desinfektionsmittel befüllten Probenaufnahmegefäß 2 bestückt. Die Halterung 3 wird dabei lediglich so weit in die Probensonde 1 eingeführt, dass sich der Stift 37 in der Stufe 19a der Nut 16 befindet. In dieser Position sticht die Nadel 13 bereits durch das Septum 21, sodass die Nadel durch das in dem Probenaufnahmegefäß 2 enthaltene Desinfektionsmittel bereits desinfiziert bzw. sterilisiert wird. Das Ventil der Probensonde 1 ist in diesem Zustand jedoch geschlossen, wie es beispielsweise in Figur 5A dargestellt ist.

Im Falle, dass eine Probe genommen werden soll, wird das Desinfektionsgefäß entfernt und die Halterung mit einem geeigneten Probenaufnahmegefäß 2 bestückt. Diese wird dann wiederum in die Probensonde 1 eingeführt, wobei zuerst eine axiale Bewegung des Stiftes in der Nut 16 und anschließend eine seitliche Bewegung des Stiftes 37 in der Nut 16 längs des Absatzes 19a und anschließend wieder eine axiale Bewegung des Stiftes 37 in der Nut 16 bis zum Anschlag 19b durchgeführt wird. In letzterer Position durchsticht die Nadel 13 das Septum 21 des Probenaufnahmegefäßes 2 und der Ventilzylinder 18 ist, wie in Figur 5B dargestellt, verschoben, um das Ventil 12 zu öffnen.

Damit kann Probe in das Probenaufnahmegefäß 2 einströmen.

Durch die bajonettartige Verriegelung der Halterung 3 in der Probensonde 1 ist gewährleistet, dass die unterschiedlichen Positionen der Probenaufnahmegefäße 2 für die unterschiedlichen Funktionen auch von nur gering geschultem Personal sicher und zuverlässig eingestellt werden können.

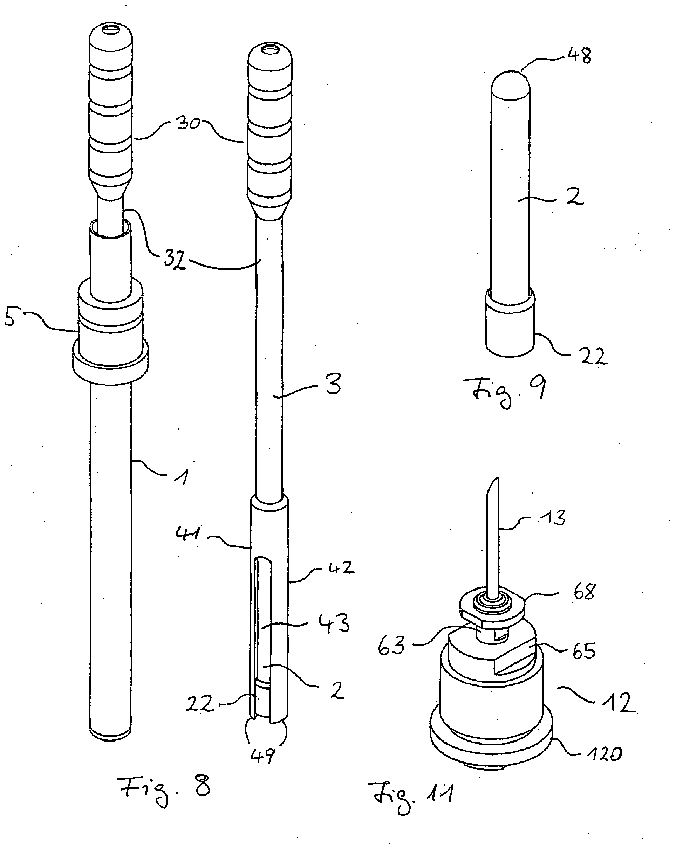

Figur 8 zeigt ein erfindungsgemäßes Probennahmesystem, bei welchem das Probenaufnahmegefäß 2 an der Halterung 3 durch eine Halteklammer 41 gehalten wird. Das rechte Teilbild zeigt die Halterung 3 mit der Halteklammer 41 außerhalb der Probensonde 1 und das linke Teilbild zeigt das gleiche System eingeführt in die Probensonde 1. Die Halteklammer 41 weist einen zylindrischen Grundkörper 42 mit schlitzförmigen Fräsungen 43 in den Seiten der Aufnahmebohrung 44 für das Probengefäß 2 auf.

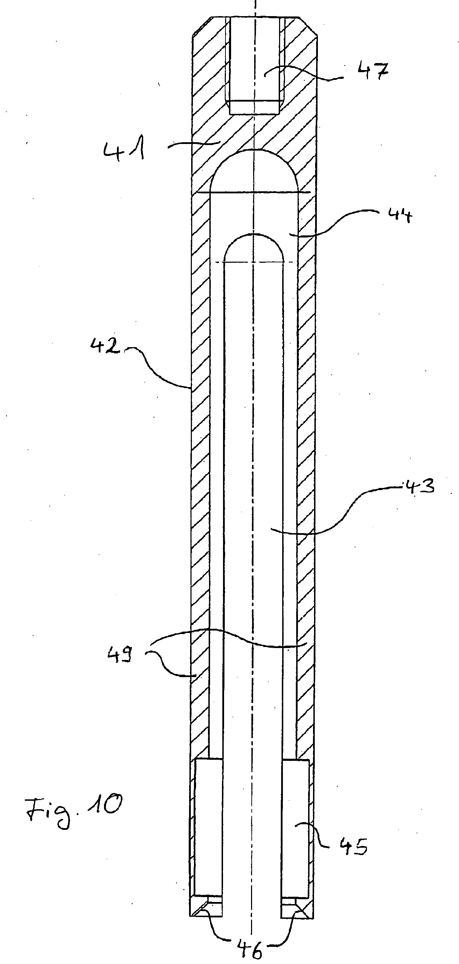

Figur 10 zeigt vergrößert den Schnitt durch eine solche Halteklammer 41. Die Bohrung 44 ist zur Aufnahme der Kappe 22 des Probengefäßes 2 mit einer entsprechenden Hinterschneidung 45 versehen. Ein entsprechendes Probengefäß 2 ist in Figur 9 gezeigt. Zum leichteren Einführen des Probengefäßes 2 in die Aufnahmebohrung 44 sind an der Aufnahmeöffnung Phasen 46 angebracht. Die Montage der Halteklammer 41 an der Halterung 3 kann über eine Gewindebohrung 47 erfolgen. Soll nun eine Probe aus einem Bioreaktor entnommen werden, so wird ein Probengefäß 2 mit dem kugelförmigen Ende 48 voraus in die Halteklammer 41 gesteckt. Dabei werden die beiden Hälften 49 der Halteklammer 41 auseinander gedrückt, bis die Kappe 22 des Probengefäßes 2 in der Hinterschneidung 45 einrastet. In diesem Zustand werden die beiden Hälften 49 der Klammer 41 leicht konisch nach außen gedrückt, erst durch die Einführung in die Sonde 1 wird die Kappe 22 des Gefäßes 2 wieder zusammengepresst und die Klammer 41 nimmt eine zylindrische Form an. Da die Klammerhälften 49 in der Sonde 1 in radialer Richtung nicht ausweichen können, wird das Gefäß sicher in der Klammer 41 gehalten und ein Herausgleiten verhindert. Zur Entnahme des Probengefäßes 2 müssen die beiden Klammerhälften 49 manuell leicht aufgebogen werden, wodurch das Probengefäß 2 leicht entnommen werden kann.

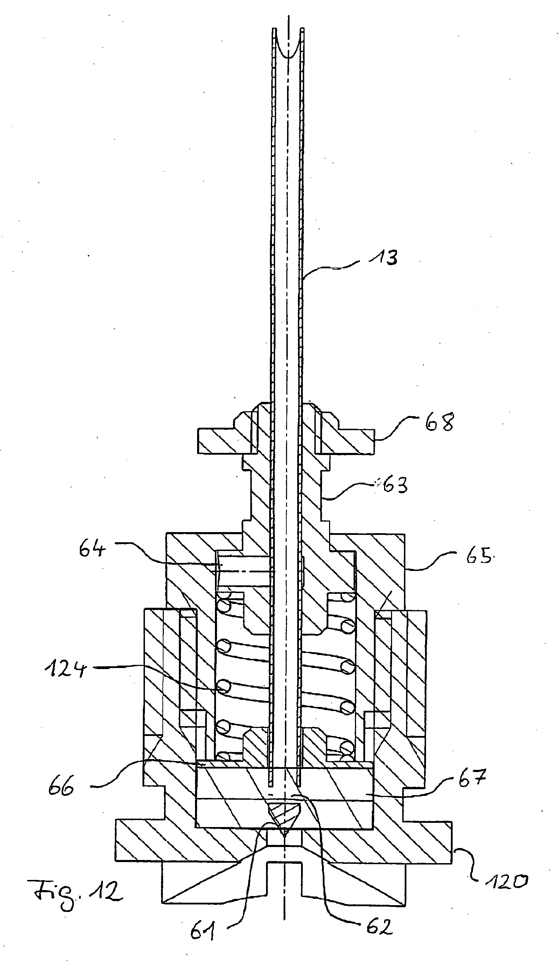

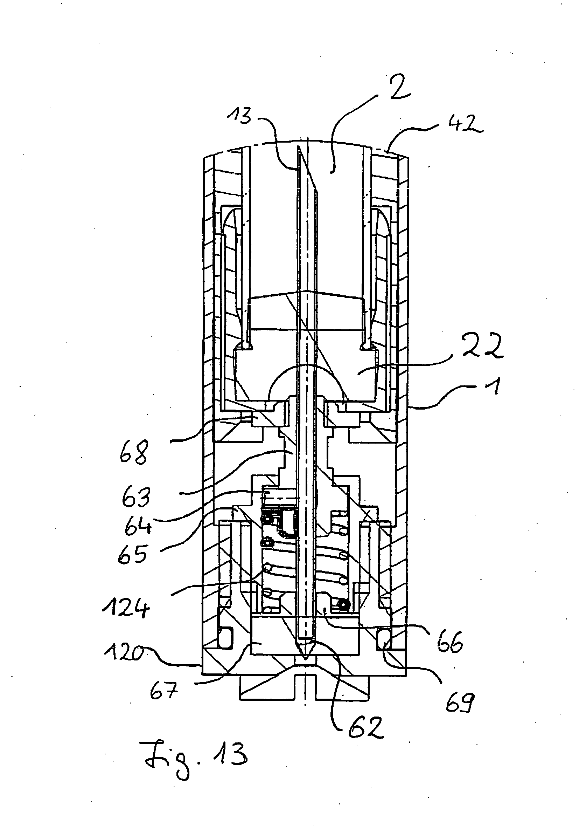

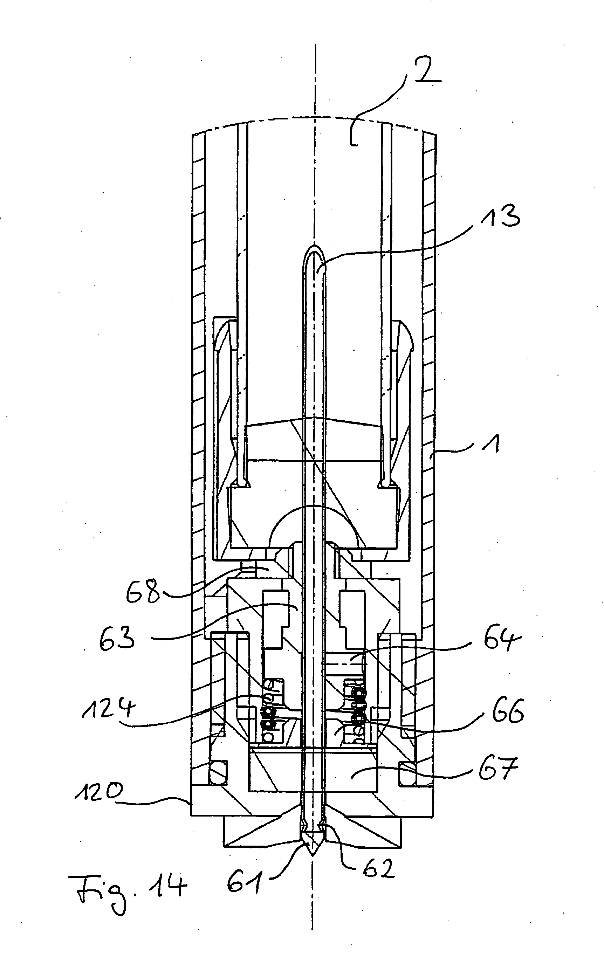

Die Figuren 11, 12, 13 und 14 zeigen eine erfindungsgemäße Ausführungsform des Ventils 12. Dabei ist in Figur 11 eine Außenansicht des Ventils zu sehen, Figur 12 zeigt einen Schnitt durch das erfindungsgemäße Ventil und die Figuren 13 und 14 zeigen das erfindungsgemäße Ventil, wenn es in die Probennahmesonde 1 eingebaut ist und mit einem Probennahmegefäß 2 verbunden ist. Hierbei stellt Figur 13 den geschlossenen Zustand und Figur 14 den geöffneten Zustand des Ventils dar. Die Montage dieses Ventils erfolgt dadurch, dass die Kanüle 13 im Lichtbogen an einem Ende zugeschweißt, anschließend eine Spitze 61 angeschliffen wird und knapp oberhalb der Spitze mit einer Querbohrung 62 versehen wird. Die Kanüle 13 wird in den Nadelkörper 63 eingelötet. Dazu befindet sich im Nadelkörper 63 eine Bohrung 64 zum Einbringen des Lots. Die Arretierung 65 wird bis zum Anschlag in den Basiskörper 120 eingeschraubt und spannt über den Nadelkörper die Feder 124 auf die Druckplatte 66 und das Septum 67 vor. Anschließend kann der Anschlag 68 auf den Nadelkörper 63 aufgeschraubt werden. Das montierte Ventil 12 wird von unten in das Rohr 1 der Probennahmesonde eingeschraubt und mit einem O-Ring 69 abgedichtet.

Wie in Figur 13 zu erkennen ist, drückt im geschlossenen Zustand die Feder 124 die Kanüle 13 über den Nadelkörper 63 in Richtung des Probengefäßes 2 und hält damit die Querbohrung 62 im Septum 67. Aufgrund der Vorspannung des Septums 67 durch die Arretierung 65 dichtet das Septum 67 die Querbohrung 62 der Nadel 13 zuverlässig ab. Ein in die Probennahmesonde 1 eingeführtes Probengefäß 2 kann gegen einen geringen Widerstand auf die Nadel 13 gedrückt werden, bis die Kappe 22 des Gefäßes 2 auf dem Anschlag 68 zu liegen kommt. Dabei ist ein deutlicher Druckpunkt zu spüren. Die Vorspannung der Feder 124 verhindert ein Öffnen des Ventils 12. Die Abdichtung der Nadelquerbohrung 62 durch das Septum 67 erhält das Vakuum im Probennahmegefäß 2.

Wie in Figur 14 zu erkennen, wird durch Erhöhung des Druckes gegen die Druckfeder 124 die Nadelspitze 61 aus dem Septum 67 bis zum Erreichen eines zweiten Druckpunktes geschoben, wodurch sie frei im anzusaugenden Medium liegt. Dadurch wird eine durchgängige Verbindung zwischen dem Reaktorinneren und dem Probengefäßinneren 2 geschaffen und durch das Vakuum kann eine Probe in das Probennahmegefäß 2 gesaugt werden. Durch Verringerung des Drucks auf das Ventil 12 zieht die Feder 124 die Kanüle 13 zurück in das Septum 67 und schließt damit das Ventil 12. Das Probennahmegefäß 2 kann anschließend gegen einen geringen Widerstand von der Nadel 13 abgezogen werden.

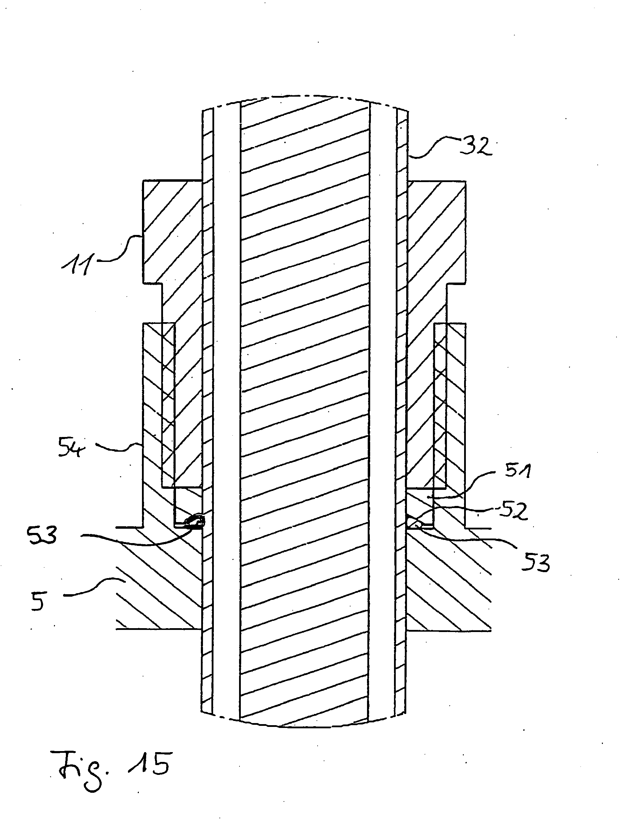

Figur 15 zeigt die Möglichkeit einer abdichtenden Deckelverschraubung. Hierbei wird auf das Rohr 32 der Probennahmesonde 1 ein Ring 51 aufgeschrumpft. Dieser verfügt über eine innen liegende Fase 52. In diese wird ein O-Ring 53 gelegt. Mit Hilfe der Überwurfmutter 11 wird der Ö-Ring 53 gegen den Einschraubbecher 54 gepresst und dichtet dadurch gegen das Rohr 32 und den Deckel 5 ab. Durch die Verwendung eines aufgeschrumpften Ringes 51 können in der Herstellung sehr leicht unterschiedlich lange Probennahmesonden 1 gefertigt werden.

Zusammenfassend lässt sich feststellen, dass im Gegensatz zur bisher durchgeführten manuellen Probennahme mit einem Tauchrohr bei der vorliegenden Erfindung durch die Probennahmeposition direkt im Reaktor das Totvolumen auf wenige µL verringert werden kann. Dies ist besonders für Laborreaktoren aus Glas, die nur von oben zugänglich sind, relevant. Außerdem wird die praktische Durchführung einer Probennahme mit dem erfindungsgemäßen System stark vereinfacht. Damit werden manuelle Fehler, die bei der üblichen Probennahme nach dem Stand der Technik häufig auftreten, vermieden, sodass die Probennahme zuverlässiger, reproduzierbarer und sicherer durchführbar ist.

## Patentansprüche

1. Probennahmesystem für fluide Proben mit
a) einem Probenaufnahmegefäß (2) zur Aufnahme der Probe, das mit einem Septum (21) verschlossein ist und
b) einer Halterung (3) für das Probenaufnahmegefäß (2) zur Aufnahme und Halterung des Probenaufnahmegefäßes (2), sowie
c) einer Probensonde (1) zum Eintauchen in ein Fluidvolumen und zur Entnahme einer fluiden Probe aus dem Fluidvolumen,
wobei die Probensonde (1) als Hohlvolumen (10) ausgebildet ist, das an einem Ende eine erste Öffnung zur Einführung der Halterung (3) und des Probenaufnahmegefäßes (2) aufweist und an einem anderen Ende durch ein mit einem in den Innenraum der Probensonde (1) vorstehenden Hohlnadel (13) versehenes erstes Ventil (12) verschlossen ist, das durch Kontakt mit der Halterung (3) und/oder dem Probenaufnahmegefäß (2) öffenbar ist und dann den Außenraum der Probensonde (1) mit dem Innenvolumen der Hohlnadel (13) verbindet.

2. Probennahmesystem nach dem vorhergehenden Anspruch, **dadurch gekennzeichnet, dass** die Probensonde (1), die Halterung (3) und das Probenaufnahmesystem (2) zylinderförmig ausgebildet sind.

3. Probennahmesystem nach einem der vorhergehenden Ansprüche, **dadurch gekennzeichnet, dass** die erste Öffnung der Probensonde (1) mittels eines Befestigungselementes (11) einem an Reaktor befestigbar ist.

4. Probennahmesystem nach einem der vorhergehenden Ansprüche, **dadurch gekennzeichnet, dass** das Probeaufnahmegefäß (2) teilweise mit einem Reagenz gefüllt ist.

5. Probennahmesystem nach einem der vorhergehenden Ansprüche, **dadurch gekennzeichnet, dass** das Probeaufnahmegefäß (2) im Inneren einen geringeren Druck als das Fluidvolumen aufweist bzw. der nicht mit Flüssigkeit gefüllte Teil des Probenaufnahmegefäßes (2) zumindest teilweise evakuiert ist.

6. Probennahmesystem nach einem der vorhergehenden Ansprüche, **dadurch gekennzeichnet, dass** in der Probensonde (1) eine Feder (125) angeordnet ist, die auf ein eingeführtes Probeaufnahmegefäß (2) und/oder eine eingeführte Halterung (3) eine rückwirkende Kraft ausübt.

7. Probennahmesystem nach einem der vorhergehenden Ansprüche, **dadurch gekennzeichnet, dass** das Probeaufnahmegefäß (2) ein zumindest teilweise evakuiertes Reagenzglas ist.

8. Probennahmesystem nach einem der vorhergehenden Ansprüche, **dadurch gekennzeichnet, dass** die Halterung (3) ein Halteelement (32) aufweist, das als Hohlrohr (32) ausgebildet ist mit einem Innendurchmesser, der größer ist als ein Außendurchmesser des Probeaufnahmegefäßes (2).

9. Probennahmesystem nach dem vorhergehenden Anspruch, **dadurch gekennzeichnet, dass** an dem Halteelement (32) mindestens ein Federelement (33) angeordnet ist, das über eine Ausnehmung (35) in der Wandung des Hohlrohres (32) in den Innenraum des Hohlrohres (32) eingreift.

10. Probennahmesystem nach dem vorhergehenden Anspruch, **dadurch gekennzeichnet, dass** das Federelement (33) eine Blattfeder ist.

11. Probennahmesystem nach dem vorhergehenden Anspruch, **dadurch gekennzeichnet, dass** die Blattfeder (33) ein erstes, fest an der Außenseite des Hohlrohres (32) angeordnetes Ende und ein zweites, freies Ende aufweist wobei das freie Ende zumindest teilweise in die Ausnehmung (35) eingreift.

12. Probennahmesystem nach einem der Ansprüche 9 bis 11, **dadurch gekennzeichnet, dass** im Bereich der Ausnehmung (32) in der Wandung des Hohlrohres (32) oder innerhalb des Hohlrohres (32) ein Dichtring (34) angeordnet ist.

13. Probennahmesystem nach einem der vorhergehenden Ansprüche,
**dadurch gekennzeichnet, dass** die Halterung in ihrem das Probenaufnahmegefäß aufnehmenden Bereich zylinderförmig ist, wobei der das Probenaufnahmegefäß aufnehmende Bereich von seinem Rand entlang zumindest eines Teils seiner Länge auf gegenüberliegenden Seiten eingeschnitten ist.

14. Probennahmesystem nach einem der vorhergehenden Ansprüche, **dadurch gekennzeichnet, dass** die Probensonde (1) und die Halterung (3) komplementäre Anschlagselemente (16, 37) aufweisen, die vorbestimmte Eindringtiefen der Halterung (3) in die Probensonde (1) festlegen.

15. Probennahmesystem nach einem der vorhergehenden Ansprüche, **dadurch gekennzeichnet, dass** die Halterung (3) einen Stift (37) aufweist und die Probensonde (1) auf ihrer Innenseite eine gestufte Nut zum Eingriff und zur Führung des Stiftes (37) aufweist, wobei die Nut (16) in axialer Richtung der Probensonde mindestens eine erste Stufe (17) und einen zweite Anschlag (18) aufweist, und
wobei bei Anlage des Stiftes (37) an die erste Stufe (17) das Septum (21) eines in der Halterung (3) befindlichen Probenaufnahmegefäßes (2) durch die Hohlnadel (13) durchstochen ist und bei Anlage an den zweiten Anschlag (18) zusätzlich das Ventil (12) geöffnet ist.

16. Probennahmesystem nach einem der vorhergehenden Ansprüche, **dadurch gekennzeichnet, dass** die Probensonde (1) auf ihrer Innenseite einen Stift aufweist und die Halterung eine gestufte Nut zum Eingriff und zur Führung des Stiftes aufweist,
wobei die Nut in axialer Richtung der Halterung mindestens eine erste Stufe und einen zweiten Anschlag aufweist,
wobei bei Anlage des Stiftes an die erste Stufe das Septum (21) eines in der Halterung (3) befindlichen Probenaufnahmegefäßes (2) durch die Hohlnadel (13) durchstochen ist und bei Anlage an den zweiten Anschlag zusätzlich das Ventil (12) geöffnet ist.

17. Verwendung eines Probenahmesystems nach einem der vorhergehenden Ansprüche zur Probenahme aus Laborreaktoren (4), Bioreaktoren, Reaktoren aus Glas, zur Entnahme toxischer oder gesundheitsgefährdender Proben aus Reaktoren und/oder zur Entnahme von Flüssigkeiten oder Gasen.

## Claims

1. Sampling system for fluid samples having
a) a sample receiving vessel (2) for receiving the sample, which is sealed by a septum (21) and
b) a holder (3) for the sample receiving vessel (2) for receiving and holding the sample receiving vessel (2), and also
c) a sample probe (1) for dipping into a fluid volume and for taking a fluid sample from the fluid volume, the sample probe (1) being configured as a hollow volume (10) which, at one end, has a first opening for introducing the holder (3) and the sample receiving vessel (2) and, at another end, is sealed by a first valve (12) which is provided with a hollow needle (13) which projects into the interior of the sample probe (1), said valve being able to be opened by contact with the holder (3) and/or with the sample receiving vessel (2) and then connecting the exterior of the sample probe (1) to the inner volume of the hollow needle (13).

2. Sampling system according to the preceding claim, **characterised in that** the sample probe (1), the holder (3) and the sample receiving system (2) are configured cylindrically.

3. Sampling system according to one of the preceding claims, **characterised in that** the first opening of the sample probe (1) can be mounted on a reactor by means of a mounting element (11).

4. Sampling system according to one of the preceding claims, **characterised in that** the sample receiving vessel (2) is filled partially with a reagent.

5. Sampling system according to one of the preceding claims, **characterised in that** the sample receiving vessel (2) has a lower pressure in the interior than the fluid volume, or the part of the sample receiving vessel (2) which is not filled with liquid is evacuated at least partially.

6. Sampling system according to one of the preceding claims, **characterised in that** a spring (125) is disposed in the sample probe (1) and exerts a retroactive force on an introduced sample receiving vessel (2) and/or on an introduced holder (3).

7. Sampling system according to one of the preceding claims, **characterised in that** the sample receiving vessel (2) is an at least partially evacuated test tube.

8. Sampling system according to one of the preceding claims, **characterised in that** the holder (3) has a holding element (32) which is configured as a hollow tube (32) with an internal diameter which is greater than an external diameter of the sample receiving vessel (2).

9. Sampling system according to the preceding claim, **characterised in that** at least one spring element (33) is disposed on the holding element (32) and engages via a recess (35) in the wall of the hollow tube (32) in the interior of the hollow tube (32).

10. Sampling system according to the preceding claim, **characterised in that** the spring element (33) is a leaf spring.

11. Sampling system according to the preceding claim, **characterised in that** the leaf spring (33) has a first end which is disposed securely on the outside of the hollow tube (32) and a second free end, the free end engaging at least partially into the recess (35).

12. Sampling system according to one of the claims 9 to 11, **characterised in that** a sealing ring (34) is disposed in the region of the recess (32) in the wall of the hollow tube (32) or within the hollow tube (32).

13. Sampling system according to one of the preceding claims, **characterised in that** the holder is cylindrical in its region which receives the sample receiving vessel, the region of its edge which receives the sample receiving vessel being cut into along at least one part of its length on opposite sides.

14. Sampling system according to one of the preceding claims, **characterised in that** the sample probe (1) and the holder (3) have complementary stop elements (16, 37) which fix predetermined penetration depths of the holder (3) into the sample probe (1).

15. Sampling system according to one of the preceding claims, **characterised in that** the holder (3) has a pin (37) and the sample probe (1), on the inner side thereof, has a stepped groove for engagement and for guidance of the pin (37), the groove (16) having, in the axial direction of the sample probe, at least a first step (17) and a second stop (18), and
upon abutment of the pin (37) against the first step (17), the septum (21) of a sample receiving vessel (2) which is situated in the holder (3) being pierced by the hollow needle (13) and, upon abutment against the second stop (18), the valve (12) being opened in addition.

16. Sampling system according to one of the preceding claims, **characterised in that** the sample probe (1), on the inner side thereof, has a pin and the holder has a stepped groove for engagement and for guidance of the pin,
the groove having, in the axial direction of the holder, at least a first step and a second stop, upon abutment of the pin against the first step, the septum (21) of a sample receiving vessel (2) which is situated in the holder (3) being pierced by the hollow needle (13) and, upon abutment against the second stop, the valve (12) being opened in addition.

17. Use of a sampling system according to one of the preceding claims for sampling from laboratory reactors (4), bioreactors, reactors made of glass, for taking toxic or health-endangering samples from reactors and/or for removing liquids or gases.

## Revendications

1. Système de prélèvement d'échantillon pour échantillons fluides avec
a) un récipient de réception d'échantillon (2) pour recevoir l'échantillon, lequel récipient est fermé avec un septum (21) et
b) un support (3) pour le récipient de réception d'échantillon (2) pour recevoir et fixer le récipient de réception d'échantillon (2), ainsi qu'avec
c) une sonde d'échantillon (1) à immerger dans un volume de fluide et pour prélever un échantillon fluide du volume de fluide,
la sonde d'échantillon (1) étant réalisée sous la forme d'un volume creux (10) qui présente à une extrémité une première ouverture pour l'introduction du support (3) et du récipient de réception d'échantillon (2), et qui est fermé à une autre extrémité par une première soupape (12) pourvue d'une aiguille creuse (13) pénétrant à l'intérieur du volume intérieur de la sonde d'échantillon (1), laquelle soupape peut être ouverte par contact avec le support (3) et/ou avec le récipient de réception d'échantillon, et qui relie ensuite le volume extérieur de la sonde d'échantillon (1) au volume intérieur de l'aiguille creuse (13).

2. Système de prélèvement d'échantillon selon la revendication précédente, **caractérisé en ce que** la sonde d'échantillon (1), le support (3) et le système de prélèvement d'échantillon (2) sont réalisés sous la forme de cylindres.

3. Système de prélèvement d'échantillon selon l'une quelconque des revendications précédentes, **caractérisé en ce que** la première ouverture de la sonde d'échantillon (1) peut être fixée à un réacteur au moyen d'un élément de fixation (11).

4. Système de prélèvement d'échantillon selon l'une quelconque des revendications précédentes, **caractérisé en ce que** le récipient de réception d'échantillon (2) est rempli en partie avec un réactif.

5. Système de prélèvement d'échantillon selon l'une quelconque des revendications précédentes, **caractérisé en ce que** le récipient de réception d'échantillon (2) présente à l'intérieur une pression inférieure au volume de fluide ou la partie non remplie de fluide du récipient de réception d'échantillon (2) est au moins en partie mise sous vide.

6. Système de prélèvement d'échantillon selon l'une quelconque des revendications précédentes, **caractérisé en ce que** dans la sonde d'échantillon (1) est disposé un ressort (125) qui exerce une force de rappel sur un récipient de réception d'échantillon (2) introduit et/ou un support (3) introduit.

7. Système de prélèvement d'échantillon selon l'une quelconque des revendications précédentes, **caractérisé en ce que** le récipient de réception d'échantillon (2) est une éprouvette au moins en partie sous vide.

8. Système de prélèvement d'échantillon selon l'une quelconque des revendications précédentes, **caractérisé en ce que** le support (3) comporte un élément de retenue (32) qui est réalisé sous la forme d'un tube creux (32) avec un diamètre intérieur supérieur à un diamètre extérieur du récipient de réception d'échantillon (2).

9. Système de prélèvement d'échantillon selon la revendication précédente, **caractérisé en ce que** sur l'élément de retenue (32) soit disposé au moins un élément de ressort (33) qui agit dans le volume intérieur du tube creux (32), à travers un évidement (35) de la paroi du tube creux (32).

10. Système de prélèvement d'échantillon selon la revendication précédente, **caractérisé en ce que** l'élément de ressort (33) est un ressort à lame.

11. Système de prélèvement d'échantillon selon la revendication précédente, **caractérisé en ce que** le ressort à lame (33) présente une première extrémité disposée de manière fixe sur la face extérieure du tube creux (32) et une seconde extrémité libre, l'extrémité libre s'engageant au moins en partie dans l'évidement (35).

12. Système de prélèvement d'échantillon selon l'une quelconque des revendications 9 à 11, **caractérisé en ce qu'**une bague d'étanchéité (34) est disposée dans la zone de l'évidement (32) de la paroi du tube creux (32) ou à l'intérieur du tube creux (32).

13. Système de prélèvement d'échantillon selon l'une quelconque des revendications précédentes, **caractérisé en ce que** le support est de forme cylindrique dans sa zone recevant le récipient de réception d'échantillon, la zone recevant le récipient de réception d'échantillon étant entaillée le long du bord d'au moins une partie de sa longueur, sur des côtés opposés.

14. Système de prélèvement d'échantillon selon l'une quelconque des revendications précédentes, **caractérisé en ce que** la sonde d'échantillon (1) et le support (3) comportent des éléments de butée (16, 37) complémentaires qui fixent des profondeurs de pénétration prédéterminées du support (3) dans la sonde d'échantillon (1).

15. Système de prélèvement d'échantillon selon l'une quelconque des revendications précédentes, **caractérisé en ce que** le support (3) comporte une languette (37) et la sonde d'échantillon (1) une rainure étagée, sur son côté intérieur, pour l'engagement et le guidage de la languette (37), la rainure (16) présentant dans la direction axiale de la sonde d'échantillon, au moins un premier gradin (17) et une seconde butée (18), et
lorsque la languette (37) s'applique contre le premier gradin (17), le septum (21) d'un récipient de réception d'échantillon (2) se trouvant dans le support (31), est transpercé par l'aiguille creuse (13) et, lorsqu'il s'applique contre la seconde butée (18), la soupape (12) est en outre ouverte.

16. Système de prélèvement d'échantillon selon l'une quelconque des revendications précédentes, **caractérisé en ce que** la sonde d'échantillon (1) présente sur son côté intérieur une languette et le support une rainure étagée pour l'engagement et le guidage de la languette, la rainure présentant dans la direction axiale du support, au moins un premier gradin et une seconde butée, lorsque la languette s'applique contre le premier gradin, le septum (21) d'un récipient de réception d'échantillon (2) se trouvant dans le support (3), étant transpercé par l'aiguille creuse (13) et, lorsqu'elle s'applique contre la seconde butée, la soupape (12) étant en outre ouverte.

17. Utilisation d'un système de prélèvement d'échantillon selon l'une quelconque des revendications précédentes, pour le prélèvement d'échantillons de réacteurs de laboratoire (4), de bio-réacteurs, de réacteurs en verre, pour le prélèvement d'échantillons toxiques ou nocifs à la santé dans des réacteurs et/ou pour le prélèvement de liquides ou de gaz.
